# EUROPEAN PATENT SPECIFICATION

(11) **EP 1 562 945 B1**
(45) Date of publication and mention of the grant of the patent: **15.11.2006**
(21) Application number: 03770918.5
(22) Date of filing: 10.11.2003
(51) Int. Cl.: C07D 471/08, A61K 31/551, A61P 25/00, A61P 25/28, A61P 25/16

(54) **1,4-DIAZABICYCLO(3,2,2)NONANE DERIVATIVES, PREPARATION AND THERAPEUTICAL USE THEREOF**
1,4-DIZABICYCLO(3,2,2)NONANE DERIVATIVE, VERFAHREN ZUR IHRE HERSTELLUNG UND THERAPEUTICAL VERWENDUNG
DERIVES DE 1,4-DIAZABICYCLO (3,2,2)NONANE, PREPARATION ET UTILISATIONS THERAPEUTIQUES DE CES DERIVES

(30) Priority: 11.11.2002 DK 200201737
(43) Date of publication of application: 17.08.2005
(73) Proprietor: NEUROSEARCH A/S, 2750 Ballerup (DK)
(72) Inventor: PETERS, Dan, c/o NeuroSearch A/S, DK-2750 Ballerup (DK); OLSEN, Gunnar M., c/o NeuroSearch A/S, DK-2750 Ballerup (DK); NIELSEN, Elsebet Ostergaard, c/o NeuroSearch A/S, DK-2750 Ballerup (DK); JORGENSEN, Tino Dyhring, c/o NeuroSearch A/S, DK-2750 Ballerup (DK); AHRING, Philip K., c/o NeuroSearch A/S, DK-2750 Ballerup (DK)
(86) International application number: PCT/DK2003/000769
(87) International publication number: WO 2004/043960

(56) References cited:
- EP-A- 1 219 622
- WO-A-00/34279
- WO-A-01/92259
- WO-A-01/92260
- WO-A-01/92261
- WO-A-03/044019
- WO-A-03/044020
- WO-A-03/044024
- DE-A- 10 162 442

## Description

### TECHNICAL FIELD

This invention relates to novel diazabicyclic biaryl derivatives, which are found to be cholinergic ligands at the nicotinic acetylcholine receptors and modulators of the monoamine receptors and transporters. Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

### BACKGROUND ART

The endogenous cholinergic neurotransmitter, acetylcholine, exert its biological effect via two types of cholinergic receptors, the muscarinic Acetyl Choline Receptors (mAChR) and the nicotinic Acetyl Choline Receptors (nAChR).

As it is well established that muscarinic acetylcholine receptors dominate quantitatively over nicotinic acetylcholine receptors in the brain area important to memory and cognition, and much research aimed at the development of agents for the treatment of memory related disorders have focused on the synthesis of muscarinic acetylcholine receptor modulators.

Recently, however, an interest in the development of nAChR modulators has emerged. Several diseases are associated with degeneration of the cholinergic system i.e. senile dementia of the Alzheimer type, vascular dementia and cognitive impairment due to the organic brain damage disease related directly to alcoholism. Indeed several CNS disorders can be attributed to a cholinergic deficiency, a dopaminergic deficiency, an adrenergic deficiency or a serotonergic deficiency.

WO 00/34279, WO 01/92259, WO 01/92260, WO 01/92261 and EP 1219622 describe 1,4-diazabicyclo[3.3.2]nonane derivatives having affinity for nicotinic receptors.

### SUMMARY OF THE INVENTION

The present invention is devoted to the provision novel modulators of the nicotinic and/or of the monoamine receptors, which modulators are useful for the treatment of diseases or disorders related to the cholinergic receptors, and in particular the nicotinic acetylcholine receptor (nAChR), the serotonin receptor (5-HTR), the dopamine receptor (DAR) and the norepinephrine receptor (NER), and of the biogenic amine transporters for serotonin (5-HT), dopamine (DA) and norepinephrine (NE).

Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

The compounds of the invention may also be useful as diagnostic tools or monitoring agents in various diagnostic methods, and in particular for *in vivo* receptor imaging (neuroimaging), and they may be used in labelled or unlabelled form.

In its first aspect the invention provides novel diazabicyclic biaryl derivatives represented by Formula I 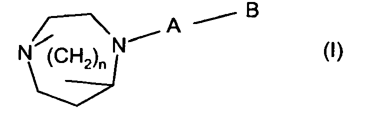 any of its enantiomers or any mixture of its enantiomers, or a pharmaceutically acceptable salt thereof, wherein
n is 1, 2 or 3; and
'A" represents a phenyl group of the formula 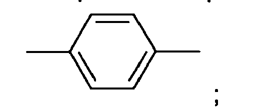 or a heterocyclic group selected from 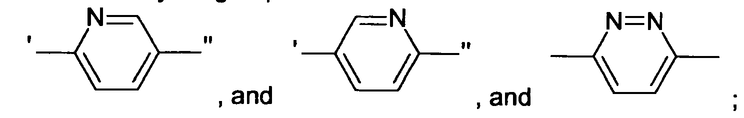 and
B represents a 5-membered aromatic monocyclic heterocyclic group selected from 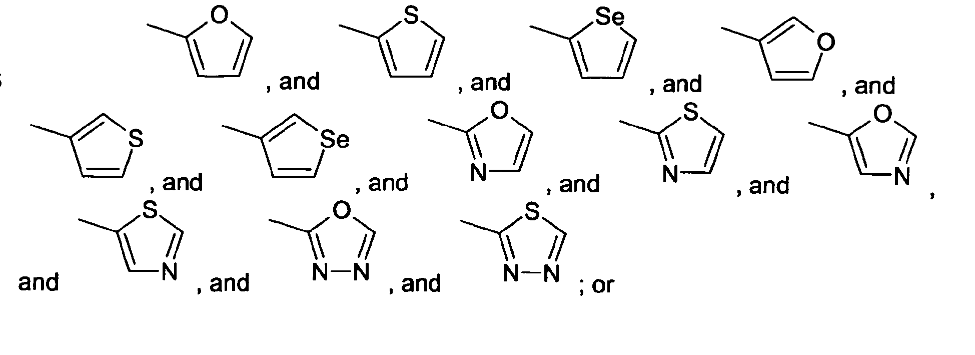
B represents a 6-membered aromatic monocyclic heterocyclic group selected from 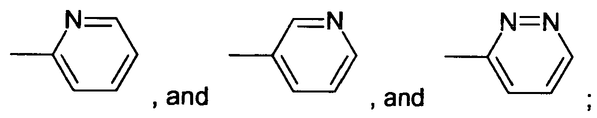 or
B represents an indol-2-yl, an indol-3-yl, an indol-5-yl or an indol-6-yl group.

In its second aspect the invention provides pharmaceutical compositions comprising a therapeutically effective amount of the diazabicyclic biaryl derivative of the invention, or a pharmaceutically-acceptable addition salt thereof, together with at least one pharmaceutically-acceptable carrier or diluent.

In a further aspect the invention relates to the use of the diazabicyclic biaryl derivative of the invention, or a pharmaceutically-acceptable addition salt thereof, for the manufacture of a pharmaceutical composition/medicament for the treatment, prevention or alleviation of a disease or a disorder or a condition of a mammal, including a human, which disease, disorder or condition is responsive to modulation of cholinergic receptors and/or monoamine receptors.

Other objects of the invention will be apparent to the person skilled in the art from the following detailed description and examples.

### DETAILED DISCLOSURE OF THE INVENTION

### Diazabicyclic Biaryl Derivative

In a first aspect novel diazabicyclic biaryl derivatives are provided. The diazabicyclic biaryl derivatives of the invention may be represented by the general Formula I 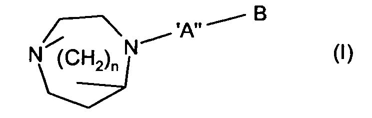 any of its enantiomers or any mixture of its enantiomers, or a pharmaceutically acceptable salt thereof, wherein
n is 1, 2 or 3; and
'A" represents a phenyl group of the formula 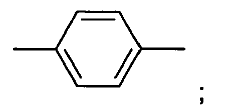
or a heterocyclic group selected from 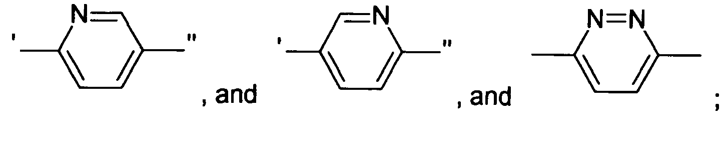 and
B represents a 5-membered aromatic monocyclic heterocyclic group selected from 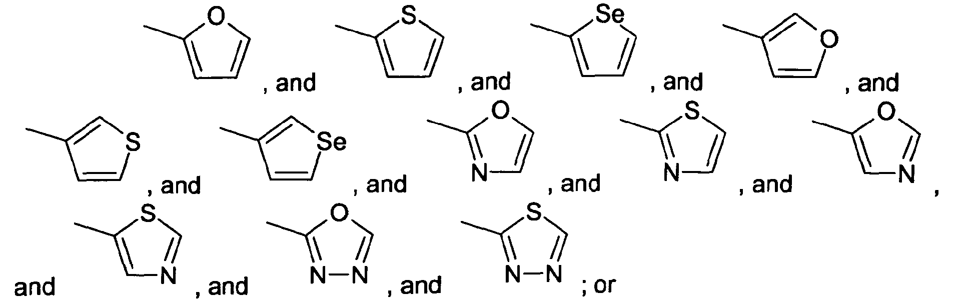
B represents a 6-membered aromatic monocyclic heterocyclic group selected from 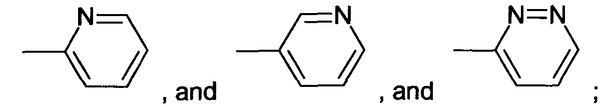 or
B represents an indol-2-yl, an indol-3-yl, an indol-5-yl or an indol-6-yl group.

In a most preferred embodiment the diazabicyclic biaryl derivative of the invention is
4-(6-Thien-3-yl-pyridazin-3-yl)-1,4-diazabicyclo[3.2.2]nonane;
4-(6-Pyridin-3-yl-pyridazin-3-yl)-1,4pyribicyclo[3.2.2]nonane;
4-(6-Selenophen-3-yl-pyridazin-3-yl)-1,4-diazabicyclo[3.2.2]nonane;
4-(6-Furan-2-yl-pyridazin-3-yl)-1,4-diazabicyclo[3.2.2]nonane;
4-(6-Thien-2-yl-pyridazin-3-yl)-1,4-diazabicyclo[3.2.2]nonane;
4-(6-Furan-3-yl-pyridazin-3-yl)-1,4-diazabicyclo[3.2.2]nonane;
4-(6-Thiazol-2-yl-pyridazin-3-yl)-1,4-diazabicyclo[3.2.2]nonane;
4-(6-Selenophen-2-yl-pyridazin-3-yl)-1,4-diazabicyclo[3.2.2]nonane;
4-(6-Indol-2-yl-pyridazin-3-yl)-1,4-diazabicyclo[3.2.2]nonane;
4-(6-Indol-3-yl-pyridazin-3-yl)-1,4-diazabicyclo[3.2.2]nonane;
4-(6-Indol-5-yl-pyridazin-3-yl)-1,4-diazabicyclo[3.2.2]nonane;
4-(6-Indol-6-yl-pyridazin-3-yl)-1,4-diazabicyclo[3.2.2]nonane;
4-(6-oxazol-2-yl-pyridazin-3-yl)-1,4-diazabicyclo[3.2.2]nonane;
4-(6-oxazol-5-yl-pyridazin-3-yl)-1,4-diazabicyclo[3.2.2]nonane;
4-(6-thiazol-5-yl-pyridazin-3-yl)-1,4-diazabicyclo[3.2.2]nonane;
4-[6-(1,3,4)-thiadiazol-2-yl-pyridazin-3-yl]-1,4-diazabicyclo[3.2.2]nonane; or
4-[6-(1,3,4)-oxadiazol-2-yl-pyridazin-3-yl]-1,4-diazabicyclo[3.2.2]nonane;
or an enantiomer or a mixture of its enantiomers, or a pharmaceutically acceptable salt thereof.

### Pharmaceutically Acceptable Salts

The diazabicyclic biaryl derivative of the invention may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate derived, the phthalate, the salicylate, the sorbate, the stearate, the succinate, the tartrate, the toluene-p-sulphonate, and the like. Such salts may be formed by procedures well known and described in the art.

Metal salts of a chemical compound of the invention include alkali metal salts, such as the sodium salt of a chemical compound of the invention containing a carboxy group.

In the context of this invention the "onium salts" of N-containing compounds are also contemplated as pharmaceutically acceptable salts. Preferred "onium salts" include the alkyl-onium salts, the cycloalkyl-onium salts, and the cycloalkylalkyl-onium salts.

Particularly preferred onium salts of the invention include those created at the N' position according to the following formula I'

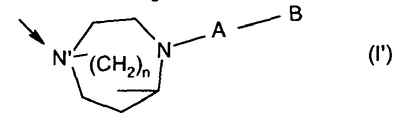

### Steric Isomers

The chemical compounds of the present invention may exist in (+) and (-) forms as well as in racemic forms. The racemates of these isomers and the individual isomers themselves are within the scope of the present invention.

Racemic forms can be resolved into the optical antipodes by known methods and techniques. One way of separating the diastereomeric salts is by use of an optically active acid, and liberating the optically active amine compound by treatment with a base. Another method for resolving racemates into the optical antipodes is based upon chromatography on an optical active matrix. Racemic compounds of the present invention can thus be resolved into their optical antipodes, e.g., by fractional crystallisation of d- or I- (tartrates, mandelates, or camphorsulphonate) salts for example.

The chemical compounds of the present invention may also be resolved by the formation of diastereomeric amides by reaction of the chemical compounds of the present invention with an optically active activated carboxylic acid such as that derived from (+) or (-) phenylalanine, (+) or (-) phenylglycine, (+) or (-) camphanic acid or by the formation of diastereomeric carbamates by reaction of the chemical compound of the present invention with an optically active chloroformate or the like.

Additional methods for the resolving the optical isomers are known in the art. Such methods include those described by *Jaques J, Collet A, & Wilen S* in "Enantiomers, Racemates, and Resolutions", John Wiley and Sons, New York (1981).

Optical active compounds can also be prepared from optical active starting materials.

### Methods of Producing Diazabicyclic Biaryl Derivatives

The diazabicyclic biaryl derivative of the invention may be prepared by conventional methods for chemical synthesis, e.g. those described in the working examples. The starting materials for the processes described in the present application are known or may readily be prepared by conventional methods from commercially available chemicals.

Also one compound of the invention can be converted to another compound of the invention using conventional methods.

The end products of the reactions described herein may be isolated by conventional techniques, e.g. by extraction, crystallisation, distillation, chromatography, etc.

### Biological Activity

The present invention relates to novel diazabicyclic biaryl derivatives, which are found to be cholinergic ligands at the nicotinic acetylcholine receptors (nAChR), and modulators of the monoamine receptors, in particular the biogenic amine transporters such as the serotonin receptor (5-HTR), the dopamine receptor (DAR) and the norepinephrine receptor (NER), and of the biogenic amine transporters for serotonin (5-HT), dopamine (DA) and norepinephrine (NE). Also preferred diazabicyclic biaryl derivatives of the invention show selective α7 activity.

In the context of this invention the term "modulator" covers agonists, partial agonists, antagonists and allosteric modulators of the receptor.

Due to their pharmacological profile the compounds of the invention may be useful for the treatment of diseases or conditions as diverse as CNS related diseases, PNS related diseases, diseases related to smooth muscle contraction, endocrine disorders, diseases related to neuro-degeneration, diseases related to inflammation, pain, and withdrawal symptoms caused by the termination of abuse of chemical substances.

In a preferred embodiment the compounds of the invention are used for the treatment of diseases, disorders, or conditions relating to the central nervous system. Such diseases or disorders includes anxiety, cognitive disorders, learning deficit, memory deficits and dysfunction, Alzheimer's disease, attention deficit, attention deficit hyperactivity disorder, Parkinson's disease, Huntington's disease, Amyotrophic Lateral Sclerosis, Gilles de la Tourette's syndrome, depression, mania, manic depression, schizophrenia, obsessive compulsive disorders (OCD), panic disorders, eating disorders such as anorexia nervosa, bulimia and obesity, narcolepsy, nociception, AIDS-dementia, senile dementia, periferic neuropathy, autism, dyslexia, tardive dyskinesia, hyperkinesia, epilepsy, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, and jet-lag.

In another preferred embodiment the compounds of the invention may be useful for the treatment of diseases, disorders, or conditions associated with smooth muscle contractions, including convulsive disorders, angina pectoris, premature labour, convulsions, diarrhoea, asthma, epilepsy, tardive dyskinesia, hyperkinesia, premature ejaculation, and erectile difficulty.

In yet another preferred embodiment the compounds of the invention may be useful for the treatment of endocrine disorders, such as thyrotoxicosis, pheochromocytoma, hypertension and arrhythmias.

In still another preferred embodiment the compounds of the invention may be useful for the treatment of neurodegenerative disorders, including transient anoxia and induced neuro-degeneration.

In even another preferred embodiment the compounds of the invention may be useful for the treatment of inflammatory diseases, disorders, or conditions, including inflammatory skin disorders such as acne and rosacea, Chron's disease, inflammatory bowel disease, ulcerative colitis, and diarrhoea.

In still another preferred embodiment the compounds of the invention may be useful for the treatment of mild, moderate or even severe pain of acute, chronic or recurrent character, as well as pain caused by migraine, postoperative pain, and phantom limb pain.

Finally the compounds of the invention may be useful for the treatment of withdrawal symptoms caused by termination of use of addictive substances. Such addictive substances include nicotine-containing products such as tobacco, opioids such as heroin, cocaine and morphine, benzodiazepines and benzodiazepine-like drugs, and alcohol. Withdrawal from addictive substances is in general a traumatic experience characterised by anxiety and frustration, anger, anxiety, difficulties in concentrating, restlessness, decreased heart rate and increased appetite and weight gain.

In this context "treatment" covers treatment, prevention, prophylactics and alleviation of withdrawal symptoms and abstinence as well as treatment resulting in a voluntary diminished intake of the addictive substance.

In another aspect, the compounds of the invention are used as diagnostic agents, e.g. for the identification and localisation of nicotinic receptors in various tissues.

### Pharmaceutical Compositions

In another aspect the invention provides novel pharmaceutical compositions comprising a therapeutically effective amount of the diazabicyclic biaryl derivative of the invention.

While a chemical compound of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the diazabicyclic biaryl derivative of the invention, or a pharmaceutically acceptable salt or derivative thereof, together with one or more pharmaceutically acceptable carriers therefore, and, optionally, other therapeutic and/or prophylactic ingredients, know and used in the art. The carrier(s) must be "acceptable" in the sense of being compatible with the other ingredients of the formulation and not harmful to the recipient thereof.

The pharmaceutical composition of the invention may be administered by any convenient route, which suits the desired therapy. Preferred routes of administration include oral administration, in particular in tablet, in capsule, in dragé, in powder, or in liquid form, and parenteral administration, in particular cutaneous, subcutaneous, intramuscular, or intravenous injection. The pharmaceutical composition of the invention can be manufactured by any skilled person by use of standard methods and conventional techniques appropriate to the desired formulation. When desired, compositions adapted to give sustained release of the active ingredient may be employed.

Further details on techniques for formulation and administration may be found in the latest edition of Remington's Pharmaceutical Sciences (Maack Publishing Co., Easton, PA).

The actual dosage depends on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be about 10 mg/kg i.v. and 100 mg/kg p.o. Preferred ranges are from about 0.1 µg/kg to about 10 mg/kg/day i.v., and from about 1 µg/kg to about 100 mg/kg/day p.o.

### EXAMPLES

The invention is further illustrated with reference to the following examples, which are not intended to be in any way limiting to the scope of the invention as claimed.

### Example 1

### Preparatory Example

All reactions involving air sensitive reagents or intermediates were performed under nitrogen and in anhydrous solvents. Magnesium sulfate was used as drying agent in the workup-procedures and solvents were evaporated under reduced pressure.

### 1,4-Diazabicyclo[3.2.2]nonane (Intermediate compound)

Was prepared according to J. Med. Chem. 1993 **36** 2311-2320 and the following slightly modified method.

To a solution of 1,4-diazabicyclo[3.2.2]nonan-3-one (15.8 g, 113 mmol) in absolute dioxane (130 ml), LiAIH₄ (4.9 g, 130 mmol) was added under argon. The mixture was refluxed for 6 hours and then allowed to reach room temperatre. To this reaction mixture, water (5 ml in 10 ml of dioxane) was added, by drops, the mixture was stirred for 0.5 hour and then filtered off via glass filter. The solvent was evaporated and the residue was distilled using a Kugelrohr apparatus at 90°C (0.1 mbar) to yield 1,4-diazabicyclo[3.2.2]nonane (11.1 g, 78%) as colourless hygroscopic material.

### 1,4-Diazabicyclof3.2.21nonan-3-one (Intermediate compound)

To a solution of 3-quinudidinone hydrochloride (45g, 278 mmol) in 90 ml of water, hydroxylamine hydrochloride (21 g, 302 mmol) and sodium acetate (CH₃COOH x 3H₂O, 83 g, 610 mmol) were added, and the mixture was stirred at 70°C for 1 hour, and then cooled to 0°C. The separated crystalline material was filtered off (without washing) and dried *in vacuo* to yield 40.0 g of oxime.

The 3-quinuclidinone oxime (40.0 g) was added by small portions during 2 hours to polyphosphoric acid* (190 g), preheated to 120°C. During the reaction the temperature of the solution was kept at 130°C. After addition of all the oxime, the solution was stirred for 20 minutes at the same temperature, then transferred to an enamelled vessel and allowed to reach room temperature. The acidic mixture was neutralized by a solution of potassium carbonate (500 g in 300 ml of water), transferred into 2000 ml flask, diluted with 300 ml of water and extracted with chloroform (3 x 600 ml). The combined organic extracts were dried with sodium sulphate, the solvent evaporated, and the solid residue dried up *in vacuo* to yield 30.0 g (77%) of a mixture of lactams.

Crystallization of the obtained mixture from 1,4-dioxane (220 ml) gave 15.8 g (40.5%) of 1,4-diazabicyclo[3.2.2]nonan-3-one as colourless large crystals with mp 211-212°C.

The filtrate was evaporated and the residue was chromatographed on a silica gel column (Merck, 9385, 230-400 mesh) with acetone as eluent. The solvent was evaporated and the residue recrystallised from ethyl etanoate to yield 1,3-diazabicyclo[3.2.2]nonan-4-one (10.2 g, 26%) as colourless fine crystals with mp 125-126°C.

### Polyphosphoric acid*

85% Orthophosphoric acid (500 g, 294 ml, 4.337 mol) was placed into a 2000 ml flask and phosphor pentoxide (750 g, 5.284 mol) was added at room temperature (ratio acid-pentoxide, 2:3). The mixture was stirred at 200-220°C for 2 hours to yield of 1250 g of polyphosphoric acid, containing 80% of P₂O₅.

### 3-Bromo-6-thien-3-yl-pyridazine (Intermediate compound)

A mixture of 3,6-dibromo-pyridazine (8.45 g, 35.5 mmol), palladacycle (0.66 g, 0.71 mmol), palladium acetate (0.16 g, 0.71 mmol), tri-*tert*-butylphosphine (0.35 ml, 1.42 mmol), aqueous potassium carbonate (2 M, 107 mmol), 1,3-propanediol (7.7 ml, 107 mmol) and 1,4-dioxane (100 ml) was stirred at reflux for 1 hour. 3-Thienyl boronic acid (5.0 g, 39.0 mmol) was added and the mixture was stirred at reflux for 7 days. Aqueous sodium hydroxide (50 ml, 1M) was added and the mixture was extracted with ethyl acetate (2 x 100 ml). Chromatography on silica gel with ethyl acetate : petroleum (1 : 3) as solvent gave the title compound. Yield 1.5 g (18%).

### 3-Chloro-6-thien-3-yl-pyridazine hydrochloric acid salt (Intermediate compound)

A mixture of 3-bromo-6-thien-3-yl-pyridazine (1.39 g, 5.8 mmol) and conc. hydrochloric acid (25 ml) was stirred at reflux for 4.5 hours. The reaction mixture was evaporated and the product was isolated in quantitative yield (1.35 g, 5.8 mmol).

### 4-(6-Bromo-pyridazin-3-yl)-1,4-diazabicyclo[3.2.2]nonane fumaric acid salt (intermediate compound)

A mixture of 3,6-dibromo-pyridazine (3.77 g, 15.85 mmol) 1,4-diazabicyclo[3.2.2]nonane (2.00 g, 15.85 mmol) and aqueous sodium hydroxide (10 ml, 4M) was stirred at 100°C for 30 minutes. The mixture was extracted with dichloromethane (3 x 20 ml). Chromatography on silica gel with dichloromethane, 10% methanol and 1% aqueous ammonia as solvent gave the title compound as an oil. Yield 0.88 g, 20%.

### 4-[6-Thien-3-yl-pyridazin-3-yl]-1,4-diazabicyclo[3.2.2]nonane fumaric acid salt (Compound 1)

A mixture of 1,4-diazabicyclo[3.2.2]nonane (0.54 g, 4.29 mmol), 3-chloro-6-thien-3-yl-pyridazine hydrochloric acid salt (1.00 g, 4.29 mmol), triethylamine (3.00 ml, 21.4 mmol) and dioxane (15 ml) was stirred at reflux for 40 hours. Aqueous sodium hydroxide (1 M, 25 ml) was added and the mixture was extracted twice with ethyl acetate (2 x 20 ml). Chromatography on silica gel with dichloromethane, 10% methanol and 1% aqueous ammonia as solvent gave the title compound as an oil. The corresponding salt was obtained by addition of a diethyl ether and methanol mixture (9 : 1) saturated with fumaric acid. Yield 0.11 g, 9%. Mp 153.7°C.

### Method A

### 4-(6-Pyridin-3-yl-pyridazin-3-yl)-1,4-diazabicyclo[3.2.2]nonane fumaric acid salt (Compound 2)

A mixture of 4-(6-bromo-pyridazin-3-yl)-1,4-diazabicyclo[3.2.2]nonane (0.49 g, 1.7 mmol), diethyl-3-pyridylborane (0.38 g, 2.6 mmol), aqueous potassium carbonate (2.6 ml, 2M), palladium(0)tetrakistriphenylphosphine (59 mg, 0.051 mmol), 1,3-propandiol (0.37 ml, 5.1 mmol) and dioxane (5 ml) was stirred at reflux for 15 hours. The mixture was evaporated. Aqueous sodium hydroxide (10 ml, 4M) was added. The mixture was extracted with dichloromethane (3 x 20 ml). Chromatography on silica gel with dichloromethane, 10% methanol and 1% aqueous ammonia as solvent gave the title compound as an oil. Yield 0.10 g, 21%.

The corresponding salt was obtained by addition of a diethyl ether and methanol mixture (9 : 1) saturated with fumaric acid. Mp 170.2-171.6°C.

### Method B

### 4-(6-Selenophen-3-yl-pvridazin-3-yl)-1,4-diazabicyclo[3.2.2]nonane fumaric acid salt (Compound 3)

A mixture of 4-(6-bromo-pyridazin-3-yl)-1,4-diazabicyclo[3.2.2]nonane (1.1 g, 3.9 mmol), 3-trimethylstannylselenophene (2.3 g, 7.8 mmol), PdCl₂(PPh₃)₂ (82 mg, 0.11 mmol) and DMF (1 ml) was stirred at 100°C for 15 hours. Aqueous sodium hydroxide (10 ml, 4M) was added. The mixture was extracted with dichloromethane (3 x 20 ml). Chromatography on silica gel with dichloromethane, 10% methanol and 1% aqueous ammonia as solvent gave the title compound as an oil. Yield 0.14 g, 11 %.

The corresponding salt was obtained by addition of a diethyl ether and methanol mixture (9 : 1) saturated with fumaric acid. Mp 181.2-182.2°C.

### 4-(6-Thien-2-yl-pyridazin-3-yl)-1,4-diazabicyclo[3.2.2]nonane fumaric acid salt (Compound 4)

Was prepared according to Method B from 2-trimetylstannylthiophene. Mp 185.5-187.4°C.

### 4-(6-Selenophen-2-yl-pyridazin-3-yl)-1,4-diazabicyclo[3.2.2]nonane fumaric acid salt (Compound 5)

Was prepared according to Method B from 2-trimetylstannylselenophene. Mp 194.7-195.9°C.

### 4-(6-Furan-2-yl-pyridazin-3-yl)-1,4-diazabicvclo[3.2.2]nonane fumaric acid salt (Compound 6)

Was prepared according to Method B from 2-trimetylstannylfuran. Mp 155.7-156.1°C.

### 4-(6-Furan-3-yl-pyridazin-3yl)-1,4-diazabicyclo[3.2.2]nonane fumaric acid salt (Compound 7)

Was prepared according to Method B from 3-trimetylstannylfuran. Mp 116.9-119.0°C.

### 4-(6-Thiazol-2-yl-pyridazin-3-yl)-1,4-diazabicyclo[3.2.2]nonane fumaric acid salt (Compound 8)

Was prepared according to Method B from 2-thiazolylzinc chloride. Mp 175.2-179.2°C.

In analogy herewith the following compounds are prepared:
4-(5-Thien-2-yl-pyrrol-2-yl)-1,4-diazabicyclo[3.2.2]nonane fumaric acid salt (Compound 9);
4-(5-Thien-2-yl-N-methyl-pyrrol-2-yl)-1,4-diazabicyclo[3.2.2]nonane fumaric acid salt (Compound 10);
4-(5-Thien-2-yl-thiazol-2-yl)-1,4-diazabicyclo[3.2.2]nonane fumaric acid salt (Compound 11);
4-(2-Thien-2-yl-thiazol-5-yl)-1,4-diazabicyclo[3.2.2]nonane fumaric acid salt (Compound 12);
4-(5-Thien-2-yl-furan-2-yl)-1,4-diazabicyclo[3.2.2]nonane fumaric acid salt (Compound 13);
4-(5-Thien-2-yl-thien-2-yl)-1,4-diazabicyclo[3.2.2]nonane fumaric acid salt (Compound 14);
4-(5-Thien-2-yl-imidazol-2-yl)-1,4-diazabicyclo[3.2.2]nonane fumaric acid salt (Compound 15);
4-(5-Thien-2-yl-*N*-methyl-imidazol-2-yl)-1,4-diazabicyclo[3.2.2]nonane fumaric acid salt (Compound 16);
4-(2-Thien-2-yl-imidazol-5-yl)-1,4-diazabicyclo[3.2.2]nonane fumaric acid salt (Compound 17);
4-(2-Thien-2-yl-*N*-methyl-imidazol-5-yl)-1,4-diazabicyclo[3.2.2]nonane fumaric acid salt (Compound 18);
4-(5-Thien-2-yl-oxazol-2-yl)-1,4-diazabicyclo[3.2.2]nonane fumaric acid salt (Compound 19);
4-(2-Thien-2-yl-oxazol-5-yl)-1,4-diazabicyclo[3.2.2]nonane fumaric acid salt (Compound 20);
4-(5-Thien-2-yl-isoxazol-3-yl)-1,4-diazabicyclo[3.2.2]nonane fumaric acid salt (Compound 21);
4-(5-Thien-2-yl-isothiazol-3-yl)-1,4-diazabicyclo[3.2.2]nonane fumaric acid salt (Compound 22);
4-(3-Thien-2-yl-isoxazol-5-yl)-1,4-diazabicyclo[3.2.2]nonane fumaric acid salt (Compound 23);
4-(3-Thien-2-yl-isothiazol-5-yl)-1,4-diazabicyclo[3.2.2]nonane fumaric acid salt (Compound 24);
4-(6-Indol-2-yl-pyridazin-3-yl)-1,4-diazabicyclo[3.2.2]nonane fumaric acid salt (Compound 25);
4-(6-Indol-3-yl-pyridazin-3-yl)-1,4-diazabicyclo[3.2.2]nonane fumaric acid salt (Compound 26);
4-(6-Indol-5-yl-pyridazin-3-yl)-1,4-diazabicyclo[3.2.2]nonane fumaric acid salt (Compound 27);
4-(6-Indol-6-yl-pyridazin-3-yl)-1,4-diazabicyclo[3.2.2]nonane fumaric acid salt (Compound 28);
4-(6-oxazol-2-yl-pyridazin-3-yl)-1,4-diazabicyclo[3.2.2]nonane fumaric acid salt (Compound 29);
4-(6-oxazol-5-yl-pyridazin-3-yl)-1,4-diazabicyclo[3.2.2]nonane fumaric acid salt (Compound 30);
4-(6-thiazol-5-yl-pyridazin-3-yl)-1,4-diazabicyclo[3.2.2]nonane fumaric acid salt (Compound 31);
4-[6-(1,3,4)-thiadiazol-2-yl-pyridazin-3-yl]-1,4-diazabicyclo[3.2.2]nonane fumaric acid salt (Compound 32); and
4-[6-(1,3,4)-oxadiazol-2-yl-pyridazin-3-yl]-1,4-diazabicyclo[3.2.2]nonane fumaric acid salt (Compound 33).

### Example 2

### In vitro Inhibition of ³H-α-Bungarotoxine Binding in Rat Brain

In this example the affinity of the compounds of the invention for binding to α₇-subtype of nicotinic receptors is determined.

α-Bungarotoxine is a peptide isolated from the venom of the Elapidae snake *Bungarus multicinctus.* It has high affinity for neuronal and neuromuscular nicotinic receptors, where it acts as a potent antagonist.

³H-α-Bungarotoxine labels nicotinic acetylcholine receptors formed by the α₇ subunit isoform found in brain and the α₁ isoform in the neuromuscular junction.

### Tissue preparation

Preparations are performed at 0-4°C. Cerebral cortices from male Wistar rats (150-250 g) are homogenised for 10 seconds in 15 ml of 20 mM Hepes buffer containing 118 mM NaCl, 4.8 mM KCl, 1.2 mM MgSO₄ and 2.5 mM CaCl₂ (pH 7.5) using an Ultra-Turrax homogeniser. The tissue suspension is subjected to centrifugation at 27,000 x g for 10 minutes. The supernatant is discarded and the pellet is washed twice by centrifugation at 27,000 x g for 10 minutes in 20 ml of fresh buffer, and the final pellet is then re-suspended in fresh buffer containing 0.01 % BSA (35 ml per g of original tissue) and used for binding assays.

### Assay

Aliquots of 500 µl of homogenate are added to 25 µl of test solution and 25 µl of ³H-α-bungarotoxine (2 nM, final concentration) and mixed and incubated for 2 hours at 37°C. Non-specific binding is determined using (-)-nicotine (1 mM, final concentration). After incubation, the samples are added 5 ml of ice-cold Hepes buffer containing 0.05% PEI and poured directly onto Whatman GF/C glass fibre filters (pre-soaked in 0.1 % PEI for at least 6 hours) under suction, and immediately washed with 2 x 5 ml ice-cold buffer.

The amount of radioactivity on the filters is determined by conventional liquid scintillation counting. Specific binding is total binding minus non-specific binding.

The test value is given as an IC₅₀ (the concentration of the test substance which inhibits the specific binding of ³H-α-bungarotoxin by 50%).

The results of these experiments are presented in Table 1 below.

**Table 1**

| Inhibition of ³H-α-Bungarotoxine Binding | |
|---|---|
| **Compound No.** | **IC**_{**50**} **(µM)** |
| 1 | 0.038 |
| 4 | 0.044 |
| 5 | 0.0065 |
| 7 | 0.022 |

## Claims

1. A diazabicyclic biaryl derivative represented by Formula I 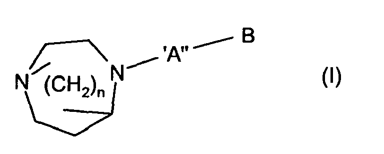 any of its enantiomers or any mixture of its enantiomers, or a pharmaceutically acceptable salt thereof, wherein
n is 1, 2 or 3; and
'A" represents a phenyl group of the formula 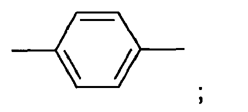 or a heterocyclic group selected from 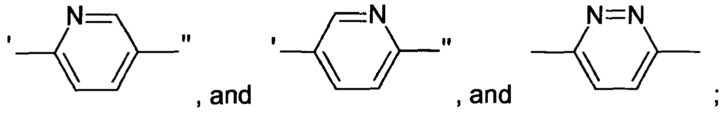 and
B represents a 5-membered aromatic monocyclic heterocyclic group selected from 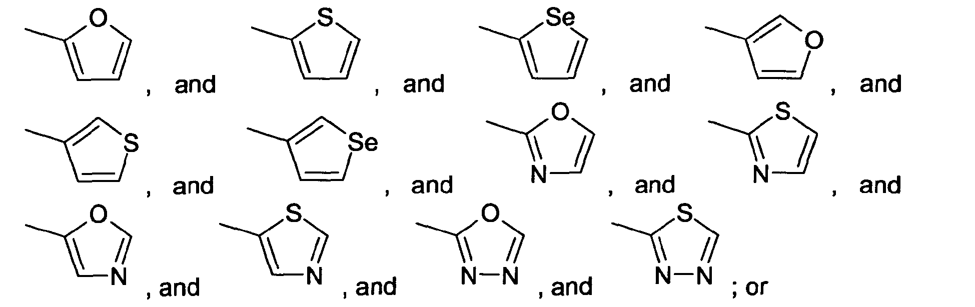
B represents a 6-membered aromatic monocyclic heterocyclic group selected from 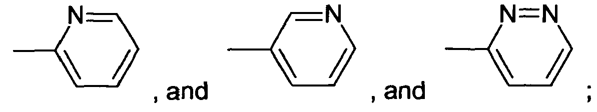 or
B represents an indol-2-yl, an indol-3-yl, an indol-5-yl or an indol-6-yl group.

2. The diazabicyclic biaryl derivative of claim 1, which is
4-(6-Thien-3-yl-pyridazin-3-yl)-1,4-diazabicyclo[3.2.2]nonane;
4-(6-Pyridin-3-yl-pyridazin-3-yl)-1,4-diazabicyclo[3.2.2]nonane;
4-(6-Selenophen-3-yl-pyridazin-3-yl)-1,4-diazabicyclo[3.2.2]nonane;
4-(6-Furan-2-yl-pyridazin-3-yl)-1,4-diazabicyclo[3.2.2]nonane;
4-(6- Thien-2-yl-pyridazin-3-yl)-1 ,4-diazabtcyclo[3.2.2]nonane;
4-(6-Furan-3-yl-pyridazin-3-yl)-1,4-diazabicyclo[3.2.2]nonane;
4-(6-Thiazol-2-yl-pyridazin-3-yl)-1,4-diazabicyclo[3.2.2]nonane;
4-(6-Selenophen-2-yl-pyridazin-3-yl)1,4-diazabicyclo[3.2.2]nonane;
4-(6-Indol-2-yl-pyridazin-3-yl)-1,4-diazabicyclo[3.2.2]nonane;
4-(6-Indol-3-yl-pyridazin-3-yl)-1,4-diazabicyclo[3.2.2]nonane;
4-(6-Indol-5-yl-pyridazin-3-yl)-1,4-diazabicyclo[3.2.2]nonane;
4-(6-Indol-6-yl-pyridazin-3-yl)-1,4-diazabicyclo[3.2.2]nonane;
4-(6-oxazol-2-yl-pyridazin-3-yl)-1,4-diazabicyclo[3.2.2]nonane;
4-(6-oxazol-5-yl-pyridazin-3-yl)-1,4-diazabicyclo[3.2.2]nonane;
4-(6-thiazol-5-yl-pyridazin-3-yl)-1,4-diazabicyclo[3.2.2]nonane;
4-[6-(1,3,4)-thiadiazol-2-yl-pyridazin-3-yl]-1,4-diazabicyclo[3.2.2]nonane; or
4-[6-(1,3,4)-oxadiazol-2-yl-pyridazin-3-yl]-1,4-diazabicyclo[3.2.2]nonane;
or an enantiomer or a mixture of its enantiomers, or a pharmaceutically acceptable salt thereof.

3. A pharmaceutical composition comprising a therapeutically effective amount of a diazabicyclic biaryl derivative of any of claims 1-2, or a pharmaceutically-acceptable addition salt thereof, together with at least one pharmaceutically-acceptable carrier or diluent.

4. Use of a diazabicyclic biaryl derivative of any of claims 1-2, or a pharmaceutically-acceptable addition salt thereof, for the manufacture of a pharmaceutical composition/medicament for the treatment, prevention or alleviation of a disease or a disorder or a condition of a mammal, including a human, which disease, disorder or condition is responsive to modulation of cholinergic receptors and/or monoamine receptors.

5. The use according to claim 4, wherein the disease, disorder or condition relates to the central nervous system.

6. The use according to claim 5, wherein the disease, disorder or condition is anxiety, cognitive disorders, learning deficit, memory deficits and dysfunction, Alzheimer's disease, attention deficit, attention deficit hyperactivity disorder, Parkinson's disease, Huntington's disease, Amyotrophic Lateral Sclerosis, Gilles de la Tourette's syndrome, depression, mania, manic depression, schizophrenia, obsessive compulsive disorders (OCD), panic disorders, eating disorders such as anorexia nervosa, bulimia and obesity, narcolepsy, nociception, AIDS-dementia, senile dementia, periferic neuropathy, autism, dyslexia, tardive dyskinesia, hyperkinesia, epilepsy, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, and jet-lag.

7. The use according to claim 4, wherein the disease, disorder or condition are associated with smooth muscle contractions, including convulsive disorders, angina pectoris, premature labour, convulsions, diarrhoea, asthma, epilepsy, tardive dyskinesia, hyperkinesia, premature ejaculation, and erectile difficulty.

8. The use according to claim 4, wherein the disease, disorder or condition is related to the endocrine system, such as thyrotoxicosis, pheochromocytoma, hypertension and arrhythmias.

9. The use according to claim 4, wherein the disease, disorder or condition is a neurodegenerative disorders, including transient anoxia and induced neuro-degeneration.

10. The use according to claim 4, wherein the disease, disorder or condition is an inflammatory disorder, including inflammatory skin disorders such as acne and rosacea, Chron's disease, inflammatory bowel disease, ulcerative colitis, and diarrhoea.

11. The use according to claim 4, wherein the disease, disorder or condition is mild, moderate or even severe pain of acute, chronic or recurrent character, as well as pain caused by migraine, postoperative pain, and phantom limb pain.

12. The use according to claim 4, wherein the disease, disorder or condition is associated with withdrawal symptoms caused by termination of use of addictive substances, including nicotine-containing products such as tobacco, opioids such as heroin, cocaine and morphine, benzodiazepines and benzodiazepine-like drugs, and alcohol.

## Patentansprüche

1. Diazabicyclisches Biarylderivat, das durch Formel I wiedergegeben wird 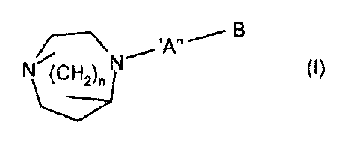 beliebige seiner Enantiomere oder ein beliebiges Gemisch von seinen Enantiomeren, oder ein pharmazeutisch annehmbares Salz davon, wobei
n 1, 2 oder 3 ist; und
'A" eine Phenylgruppe der Formel 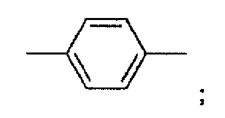 oder eine heterocyclische Gruppe, ausgewählt aus 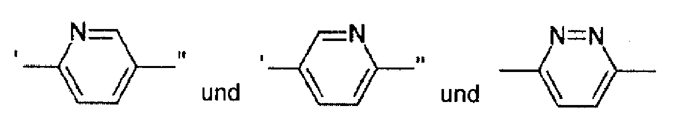 bedeutet; und
B eine 5-gliedrige aromatische monocyclische heterocyclische Gruppe, ausgewählt aus 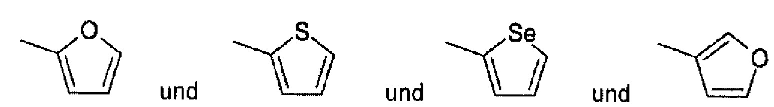 und 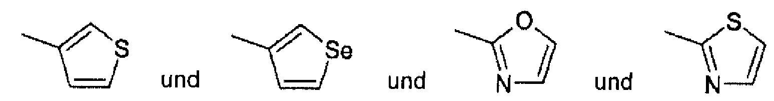 und 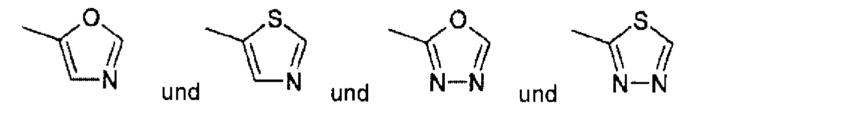 bedeutet; oder
B eine 6-gliedrige aromatische monocyclische heterocyclische Gruppe, ausgewählt aus 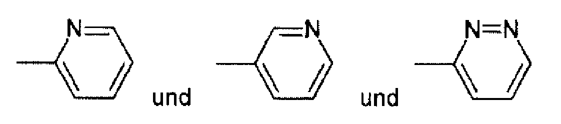 bedeutet; oder
B eine Indol-2-yl-, eine Indol-3-yl-, eine Indol-5-yl- oder eine Indol-fi-yl-gruppe bedeutet.

2. Diazabicyclisches Biarylderivat nach Anspruch 1, welches
4-(6-Thien-3-yl-pyridazin-3-yl)-1,4-diazabicyclo[3.2.2]nonan;
4-(6-Pyridin-3-yl-pyridazin-3-yl)-1,4-diazabicyclo[3.2.2]nonan;
4-(6-Selenophen-3-yl-pyridazin-3-yl)-1,4-diazabicyclo[3.2.2]nonan;
4-(6-Furan-2-yl-pyridazin-3-yl)-1,4-diazabicyclo[3.2.2]nonan;
4-(6-Thien-2-yl-pyridazin-3-yl)-1,4-diazabicyclo[3.2.2]nonan;
4-(6-Furan-3-yl-pyridazin-3-yl)-1,4-diazabicyclo[3.2.2]nonan;
4-(6-Thiazol-2-yl-pyridazin-3-yl)-1,4-diazabicyclo[3.2.2]nonan;
4-(6-Selenophen-2-yl-pyridazin-3-ya)-1,4-diazabicyclo[3.2.2]nonan;
4-(6-Indol-2-yl-pyridazin-3-yl)-1,4-diazabicyclo[3.2.2]nonan;
4-(6-Indol-3-yl-pyridazi n-3-yl)- 1 ,4-diazabicydo[3.2 .2]nonan ;
4-(6-Indol-5-yl-pyridazin-3-yl)-1,4-diazabicyclo[3.2.2]nonan;
4-(6-Indol-6-yl-pyridazin-3-yl)-1,4-diazabicyclo[3.2.2]nonan;
4-(6-Oxazol-2-yl-pyridazin-3-yl)-1,4-diazabicyclo[3.2.2]nonan;
4-(6-Oxazol-5-yl-pyridazin-3-yl)-1,4-diazabicyclo[3.2.2]nonan;
4-(6-Thiazol-5-yl-pyridazin-3-yl)-1,4-diazabicyclo[3.2.2]nonan;
4-[6-(1,3,4)-Thiadiazol-2-yl-pyridazin-3-yl]-1,4-diazabicyclo[3.2.2]nonan; oder
4-[6-(1,3,4)-Oxadiazol-2-yl-pyridazin-3-yl]-1,4-diazabicyclo[3.2.2]nonan ist, oder ein Enantiomer oder ein Gemisch von seinen Enantiomeren oder ein pharmazeutisch annehmbares Salz davon.

3. Pharmazeutische Zusammensetzung, umfassend eine therapeutisch wirksame Menge eines diazabicyclischen Biarylderivats nach einem der Ansprüche 1-2, oder eines pharmazeutisch annehmbaren Additionssalzes davon, zusammen mit wenigstens einem pharmazeutisch annehmbaren Träger oder Verdünnungsmittel.

4. Verwendung eines diazabicyclischen Biarylderivats nach einem der Ansprüche 1-2, oder eines pharmazeutisch annehmbaren Additionssalzes davon, für die Herstellung einer pharmazeutischen Zusammensetzung/eines Medikaments für die Behandlung, Verhütung oder Linderung einer Krankheit oder einer Störung oder eines Zustands bzw. Leidens eines Säugers, einschließlich eines Menschen, wobei die Krankheit, die Störung oder der Zustand bzw. das Leiden auf die Modulierung von cholinergen Rezeptoren und/oder Monoaminrezeptoren anspricht.

5. Verwendung nach Anspruch 4, wobei sich die Krankheit, die Störung oder der Zustand bzw. das Leiden auf das Zentralnervensystem bezieht.

6. Verwendung nach Anspruch 5, wobei es sich bei der Krankheit, der Störung oder dem Zustand bzw. Leiden um Angst, kognitive Störungen, Lerndefizit, Gedächtnisdefizite und -dysfunktion, Alzheimer-Krankheit, Aufmerksamkeitsdefizit, Aufmerksamkeitsdefizit-Hyperaktivitätsstörung, Parkinson-Krankheit, Chorea Huntington, amyotrophe Lateralsklerose, Gilles de la Tourette-Syndrom, Depression, Manie, manische Depression, Schizophrenie, Zwangsstörungen (OCD), Panikstörungen, Essstörungen wie Anorexia nervosa, Bulimie und Fettleibigkeit, Narkolepsie, Nozizeption, AIDS-Demenz, senile Demenz, periphere Neuropathie, Autismus, Dyslexie, tardive Dyskinesie, Hyperkinesie, Epilepsie, Bulimie, posttraumatisches Syndrom, Sozialphobie, Schlafstörungen, Pseudodemenz, Ganser-Syndrom, prämenstruelles Syndrom, Syndrom der späten Lutealphase, chronisches Ermüdungssyndrom, Mutismus, Trichotillomanie und Jetlag handelt.

7. Verwendung nach Anspruch 4, wobei die Krankheit, die Störung oder der Zustand bzw. das Leiden mit den Kontraktionen von glatten Muskeln verbunden sind, einschließlich Krampfstörungen, Angina pectoris, vorzeitiger Wehen bzw. Frühgeburt, Krämpfe, Diarrhoe, Asthma, Epilepsie, tardiver Dyskinesie, Hyperkinesie, vorzeitiger Ejakulation und Erektionsschwierigkeiten.

8. Verwendung nach Anspruch 4, wobei die Krankheit, die Störung oder der Zustand bzw. das Leiden mit dem endokrinen System zusammenhängt, wie Thyreotoxikose, Phäochromozytom, Bluthochdruck und Arrhythmien.

9. Verwendung gemäß Anspruch 4, wobei die Krankheit, die Störung oder der Zustand bzw. das Leiden eine neurodegenerative Störung ist, einschließlich vorübergehender Anoxie und induzierter Neurodegeneration.

10. Verwendung nach Anspruch 4, wobei es sich bei der Krankheit, der Störung oder dem Zustand bzw. Leiden um eine entzündliche Störung handelt, einschließlich entzündlicher Hautstörungen wie Akne und Rosazea, Morbus Crohn, entzündlicher Darmerkrankung, Colitis ulcerosa und Diarrhoe.

11. Verwendung nach Anspruch 4, wobei es sich bei der Krankheit, der Störung oder dem Zustand bzw. Leiden um milde, mäßige oder auch starke Schmerzen von akutem, chronischem oder wiederkehrendem Charakter sowie Schmerzen, die durch Migräne verursacht werden, postoperative Schmerzen und Phantomschmerzen handelt.

12. Verwendung nach Anspruch 4, wobei die Krankheit, die Störung oder der Zustand bzw. das Leiden mit Entzugssymptomen verbunden ist, die durch die Beendigung der Verwendung von süchtig machenden Substanzen, einschließlich nikotinhaltiger Produkte wie Tabak, Opioide wie Heroin, Kokain und Morphin, Benzodiazepine und Benzodiazepin-artiger Drogen bzw. Arzneimittel und Alkohol, verursacht werden.

## Revendications

1. Dérivé de biaryle diazabicyclique représenté par la formule I 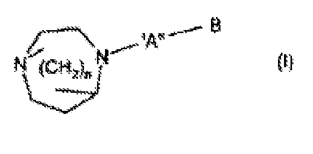 l'un quelconque de ses énantiomères ou tout mélange de ses énantiomères, ou un sel pharmaceutiquement acceptable de celui-ci, dans lequel
n vaut 1, 2 ou 3 ; et
'A" représente un groupe phényle de formule 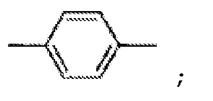 ou un groupe hétérocyclique choisi parmi 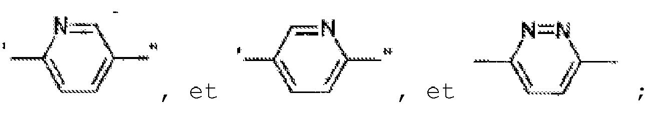 et
B représente un groupe hétérocyclique monocyclique aromatique à 5 chaînons choisis parmi 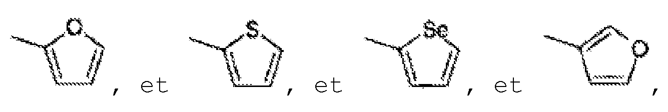 et 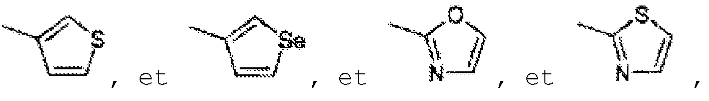 et 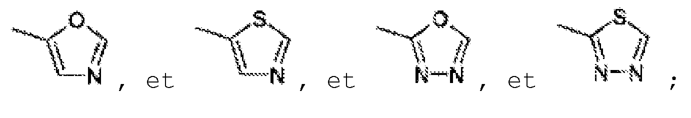 ou
B représente un groupe hétérocyclique monocyclique aromatique à 6 chaînons choisi parmi 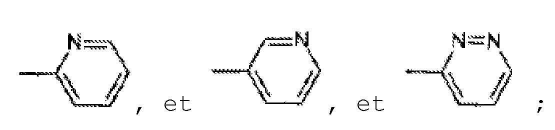 ou
B représente un groupe indol-2-yle, indol-3-yle, indol-5-yle ou indol-6-yle.

2. Dérivé de biaryle diazabicyclique selon la revendication 1, qui est
le 4-(6-thièn-3-yl-pyridazin-3-yl)-1,4-diazabicyclo[3.2.2]nonane ;
le 4-(6-pyridin-3-yl-pyridazin-3-yl)-1,4-diazabicyclo[3.2.2]nonane ;
le 4-(6-sélénophèn-3-yl-pyridazin-3-yl)-1,4-diazabicyclo[3.2.2]nonane ;
le 4-(6-furan-2-yl-pyridazin-3-yl)-1,4-diazabicyclo[3.2.2]nonane ;
le 4-(6-thièn-2-yl-pyridazin-3-yl)-1,4-diazabicyclo[3.2.2]nonane ;
le 4-(6-furan-3-yl-pyridazin-3-yl)-1,4-diazabicyclo[3.2.2]nonane ;
le 4-(6-thiazol-2-yl-pyridazin-3-yl)-1,4-diazabicyclo[3.2.2]nonane ;
le 4-(6-sélénophèn-2-yl-pyridazin-3-yl)-1,4-diazabicyclo[3.2.2]nonane ;
le 4-(6-indol-2-yl-pyridazin-3-yl)-1,4-diazabicyclo[3.2.2]nonane ;
le 4-(6-indol-3-yl-pyridazin-3-yl)-1,4-diazabicyclo[3.2.2]nonane ;
le 4-(6-indol-5-yl-pyridazin-3-yl)-1,4-diazabicyclo[3.2.2]nonane ;
le 4-(6-indol-6-yl-pyridazin-3-yl)-1,4-diazabicyclo[3.2.2]nonane ;
le 4-(6-oxazol-2-yl-pyridazin-3-yl)-1,4-diazabicyclo[3.2.2]nonane ;
le 4-(6-oxazol-5-yl-pyridazin-3-yl)-1,4-diazabicyclo[3.2.2]nonane ;
le 4-(6-thiazol-5-yl-pyridazin-3-yl)-1,4-diazabicyclo[3.2.2.]nonane;
le 4-[6-(1,3,4)-thiadiazol-2-yl-pyridazin-3-yl]-1,4-diazabicyclo[3.2.2]nonane ; ou
le 4-[6-(1,3,4)-oxadiazol-2-yl-pyridazin-3-yl]-1,4-diazabicyclo[3.2.2]nonane
ou un énantiomère ou un mélange de ses énantiomères, ou un sel pharmaceutiquement acceptable de celui-ci.

3. Composition pharmaceutique comprenant une quantité thérapeutiquement efficace d'un dérivé de biaryle diazabicyclique selon l'une quelconque des revendications 1 à 2, ou un sel d'addition pharmaceutiquement acceptable de celui-ci, conjointement avec au moins un véhicule ou diluant pharmaceutiquement acceptable.

4. Utilisation d'un dérivé de biaryle diazabicyclique selon l'une quelconque des revendications 1 à 2, ou d'un sel d'addition pharmaceutiquement acceptable de celui-ci, pour la fabrication d'une composition/médicament pharmaceutique pour le traitement, la prévention ou le soulagement d'une maladie ou d'un trouble ou d'une condition d'un mammifère, y compris un humain, la maladie, le trouble ou la condition étant sensible à une modulation des récepteurs cholinergiques et/ou des récepteurs de monoamine.

5. Utilisation selon la revendication 4, dans laquelle la maladie, le trouble ou la condition concerne le système nerveux central.

6. Utilisation selon la revendication 5, dans laquelle la maladie, le trouble ou la condition est l'anxiété, les troubles cognitifs, un déficit de l'apprentissage, des déficits et un dysfonctionnement de la mémoire, la maladie d'Alzheimer, un déficit de l'attention, un trouble déficitaire de l'attention avec hyperactivité, la maladie de Parkinson, la maladie d'Huntington, la sclérose latérale amyotrophique, le syndrome de Gilles de la Tourette, la dépression, la manie, la maniaco-dépression, la schizophrénie, les troubles obsessionnels compulsifs (TOC), la panique, les troubles de l'alimentation tels que l'anorexie mentale, la boulimie et l'obésité, la narcolepsie, la nociception, la démence liée au SIDA, la démence sénile, la neuropathie périphérique, l'autisme, la dyslexie, les dyskinésies tardives, l'hyperkinésie, l'épilepsie, la boulimie, le syndrome post-traumatique, la phobie sociale, les troubles du sommeil, la pseudo démence, le syndrome de Ganser, le syndrome pré-menstruel, le syndrome de la phase lutéale tardive, le syndrome de fatigue chronique, le mutisme, la trichotillomanie et les symptômes du décalage horaire.

7. Utilisation selon la revendication 4, dans laquelle la maladie, le trouble ou la condition sont associés à des contractions du muscle lisse, incluant les troubles convulsifs, l'angine de poitrine, l'accouchement prématuré, les convulsions, la diarrhée, l'asthme, l'épilepsie, la dyskinésie tardive, l'hyperkinésie, l'éjaculation précoce et la difficulté d'érection.

8. Utilisation selon la revendication 4, dans laquelle la maladie, le trouble ou la condition est lié au système endocrinien, tel que la thyréotoxicose, le phéochromocytome, l'hypertension et les arythmies.

9. Utilisation selon la revendication 4, dans laquelle la maladie, le trouble ou la condition est un trouble neurodégénératif, incluant l'anoxie transitoire et une neuro-dégénérescence induite.

10. Utilisation selon la revendication 4, dans laquelle la maladie, le trouble ou la condition est un trouble inflammatoire, incluant les troubles inflammatoires de la peau tels que l'acné et l'acné rosacée, la maladie de Chron, l'affection abdominale inflammatoire, la rectocolite hémorragique et la diarrhée.

11. Utilisation selon la revendication 4, dans laquelle la maladie, le trouble ou la condition est une douleur bénigne, modérée ou même sévère de caractère aigu, chronique ou récurrent, de même qu'une douleur provoquée par la migraine, une douleur post-opératoire et la douleur des membres fantômes.

12. Utilisation selon la revendication 4, dans laquelle la maladie, le trouble ou la condition est associé à des syndromes de sevrage provoqués par l'arrêt de l'utilisation de substances toxicomanogènes, incluant les produits contenant de la nicotine tels que le tabac, les opioïdes tels que l'héroïne, la cocaïne et la morphine, les benzodiazépines et les drogues de type benzodiazépine, et l'alcool.
